# EUROPEAN PATENT SPECIFICATION

(11) **EP 0 296 124 B1**
(45) Date of publication and mention of the grant of the patent: **21.12.1994**
(21) Application number: 88810415.5
(22) Date of filing: 16.06.1988
(51) Int. Cl.: A61M 1/00

(54) **Pump, particularly for enteral fluid control, cassette for pump, and method of operating pump**
Pumpe, besonders für die Kontrolle der enteral zu verabreichenden Flüssigkeit, Pumpenkassette, und Betriebsverfahren
Pompe, en particulier pour contrôle de fluide enteral, cassette pour pompe et méthode de mise en service

(30) Priority: 16.06.1987 US 62905; 20.07.1987 US 75627
(43) Date of publication of application: 21.12.1988
(73) Proprietor: FRANTZ MEDICAL DEVELOPMENT LTD., New York, N.Y. 10022 (US)
(72) Inventor: Frantz, Mark G., New York, N.Y. 10022 (US); Pavsek, Thomas J., Montor Ohio 44060 (US); Honard, Mark R., Montor Ohio 44060 (US); Sears, Lawrence M., Shaker Heights Ohio 44122 (US); Shen, Ying, Lyndhurst Ohio 44124 (US)
(74) Representative: Riederer, Conrad A., Dr.

(56) References cited:
- EP-A- 0 086 731
- WO-A-84/00691
- GB-A- 2 126 666
- US-A- 3 159 176

## Description

### DESCRIPTION

The present invention relates generally to fluid delivery systems for the administration of nutrients to humans or animals. More particularly, the invention relates to a new, improved volumetric fluid pumping system incorporating a disposable, sterilizable cassette with an internal self-actuated valve system, which may be reliably and easily inserted into, and removed from, a pump housing by relatively untrained medical personnel or unskilled patients.

### BACKGROUND

It is common medical practice to deliver nutritional fluids either enterally through the alimentary canal via a naso-gastric tube or a jejunostomy catheter, or parenterally via an intravenous catheter. Medical personnel generally utilize positive pressure pumps to deliver and regulate the flow of these nutrients.

Conventional enteral pumps are non-volumetric, positive-pressure peristaltic systems utilizing silicone rubber tubing inserts, which are in direct contact with the pump rotor, and which, in turn, are mechanically connected to the PVC tubing linking the fluid reservoir and patient to the pump. The silicone rubber insert is stretched around the rotor by means of mounting tabs which are positioned in slots or brackets on the pump face. The tubing sets also embody drip chambers which are mounted inside optic path housings in order to observe the passage of fluid drops, thereby detecting nutrient flow or the absence of flow.

The intrinsic design of these peristaltic enteral pumps results in a number of functional drawbacks, most notably: volumetric inaccuracies under many common operating conditions, unsafe operating conditions if the set is not properly mounted on the pump, and complicated set assembly techniques.

The accuracy of peristaltic pumps is greatly affected by the pressures exerted by the fluid reservoir and patient height relative to the pump, by the fluid viscosity, and by the physical properties of the silicone insert. These are non-volumetric systems as compared to positive displacement volumetric systems, such as, syringe pumps, and do not embody valving systems, which results in exogenous pressures affecting the overall flow rate. The use of expensive silicone rubber inserts has been necessary in order to maintain accuracy and avoid deterioration and splitting of the tubing over even relatively short periods. The silicone tubing must be extruded to very tight inner diameter and outer diameter tolerances and requires very precise cutting and overall length assembly within the mounting tabs.

The assembly process is further complicated because silicone is not compatible with plastic bonding techniques. This problem also creates the possibility of leakage at the attachment joints if the set is pressurized due to a downstream occlusion. The mounting of these sets has always been an acknowledged problem. Patients and medical personnel have often incorrectly mounted the sets on the pump face resulting in no flow, inaccurate flow, or in some cases, even open flow, which may cause severe patient complications. The mounting of the drip chambers within optic path housings is also an acknowledged problem, resulting in false alarm situations. The use of optic path flow detection also restricts the use of these pumps to relatively upright, motionless applications; otherwise, the fluid drops may bypass the optics beam, or may splash on the side walls and coat the walls, falsely blocking the optics beam.

Many parenteral drop flow controllers or infusion pumps are susceptible to the same types of problems as the enteral pumps. Even the more advanced, expensive, positive-pressure, volumetric syringe types perform in less than a satisfactory manner in several respects. These devices are generally complicated to operate, and allow for incorrect mounting of the disposable cartridges. These problems are documented and have even been the subject of discussion in new patent applications.

The major obstacle to proper mounting of the cartridges is the attachment of the syringe head to the pump piston mechanism. This design restriction has been documented to cause misalignment, which may even allow air or pathogens to enter into the syringe fluid chamber, as well as to cause inaccurate flow rates. It has also been reported that various valving mechanisms and alarm detector interfaces have been improperly mounted in the pump bodies.

A second major limitation of these devices is their lack of appropriateness for enteral nutritional administration. Generally these devices and the disposable cartridges are very expensive compared to enteral products, and require sophisticated trained personnel for proper operation. In addition, several cartridge designs allow for the accumulation of enteral nutrient residue in the valving inlets/outlets, which over time will adversely affect the flow rates and volume delivered.

GB-A-2 126 666 describes a pump with a disposable cassette. The cassette comprises a body with an inlet and an outlet and a bellows member defining a pumping chamber. The bellows is attached to the body by snap-on ribs. Inlet and outlet of said cassette can be opened and closed by corresponding inlet and outlet valves which are formed by resilient members. These resilient members are sealed into the body by a cover plate so that a bacterial seal is produced. For operating the valves the resilient members are stretched by operating rods so that their sealing lips disengage from the valve seats. The valves are driven by a pump drive mechanism comprising a rocker acting through plungers on the operating rods. The pump drive mechanism also has a drive member coupled to the bellows for expanding and contracting it. Synchronous operation is effected by a rotary stopper motor through cam mechanisms. The described cassette has the drawback that it requires a relatively complicated drive mechanism for actuating the valves and the bellows synchronously. This makes the pump rather expensive and unsafe in its operation.

As a result of the current state-of-the art limitations of the enteral and parenteral nutrient pump systems, a clear need exists to provide the medical community with an accurate, safe, cost-effective system which may be reliably utilized by relatively unsophisticated patients and personnel in a wide range of settings, incuding ambulatory and home care. The invention set forth below intrinsically fulfills this need.

### THE INVENTION:

Accordingly, it is among the objects of the present invention to increase the accuracy and safety of the pumping system, lower the cost of the system components and the cost of assembling them, reduce the skill and time required to set up the system for use by or on a patient, and allow the pump system to be used in an ambulatory mode.

Briefly, the present invention replaces the conventional peristaltic rotor/silicone tubing combination and syringe-type infusion pump systems with a cassette containing a compressible membrane and at least one pressure-actuated check valve. The disposable, sterilized cassette is inserted into a pump housing at a location adjacent to a reciprocating piston which drives the compressible membrane. Sensors are located within the pump chamber and are connected to a monitoring circuit in the pump housing for the purpose of alerting the user to any malfunctions or other alarm conditions. Preferably, a microprocessor is employed for monitoring purposes, setting desired flow rates, metering, and generating alarm indications.

More particularly, the present invention provides greater accuracy under a wide range of specific operating conditions, compared to many peristaltic systems, as a result of the volumetric design and the use of both an upstream and downstream valving system. The operating accuracy in the medical environment should also be improved due to the simplicity of set-up in the present invention, compared to currently available enteral and parenteral pump systems.

The present invention also provides enhanced safety due to its sealed cassette, and downstream check valve which will prevent fluid leakage, open flow of the nutrient, and the intake of air or pathogens. Safety will also be improved by the reduction in potential operator error, as the cassette simply snap-fits into place, and there are no drip chamber compartments or valve mechanisms to load. Furthermore, if the cassette is not properly locked into place, by inserting the cassette into the pump chamber past the latching mechanism, the compressible membrane will return the cassette to a position substantially outside of the pump and a pressure sensor alarm will be activated if the pump is put into the run mode. The latching mechanism also prevents accidental dislodging during use, which in a valveless peristaltic system could result in open flow.

The elimination of silicone tubing, and the inherent design of the present invention's cassette reduce the complexity of set production. The cassette is composed of all molded pieces, which are easily press-fit, sonic-welded or adhesively bonded together. There is no silicone tubing to be cut to extremely precise tolerances and there are no plastic-to-rubber joints requiring special treatment to avoid fluid leakage. Also, the pumping mechanism does not require expensive linkage systems, optic path compartments for drip chambers, or external valving actuators.

Mounting of the cassette into the circular pump chamber is an easy operation, not requiring unusual dexterity or training. The arrow-shaped cap fits into an identically shaped indentation in the pump housing, and the latching system is self-actuating. There are no drip chambers or external valves to be mounted.

The replacement of drip chamber sensors with new and improved pressure sensors and an optics sensor across the body of the cassette allow the present invention to be used in a totally ambulatory mode, i.e. the patient can walk around.

These features of the present invention fulfill well recognized needs in the medical community for a versatile, easy-to-use, safe and accurate nutritional fluid pump system. These advantages will be further obvious from the following detailed description and the accompanying drawings. The following detailed description of the preferred embodiment specifically relates to an enteral pump system; however, the invention may also easily be adapted for parenteral use by the addition of air bubble detectors or eliminators, of which many are known in the art.

### DRAWINGS:

These and other advantages of the present invention will be apparent from the drawings, in which:
Fig. 1 is a schematic view of a pumping system, showing the nutrient reservoir, the tubing leading therefrom, the bellows cassette, the pump-and-control housing into which the cassette is inserted, and the tubing from the cassette toward the patient;
Fig. 2 is a front view of the pump-and-control housing, including the novel flip-handle control setting means thereof;
Fig. 3 is a vertical cross-section through the pump housing and the cassette-receiving chamber therein;
Fig. 3A is an schematic side view of the pump housing and the irregularly-shaped cassette cap mating therewith, and showing the location of the optical sensing system components;
Fig. 4 is a longitudinal cross-section through the bellows cassette;
Fig. 5 is an end view of the bellows cassette showing the inlet and outlet tubes thereof;
Fig. 6 is cross-section along line VI-VI of Figure 4, illustrating the invention's optical empty-detection system;
Figs. 7-13 are flowcharts of the operational sequence or program of the system;
Figs. 14-16 are schematic views of the pump circuits;
Fig. 17 is a circuit diagram of a power supply circuit which may be placed in a separate charger or backpack (not shown in this application) having means for receiving the pump and for clamping itself onto a pole; and

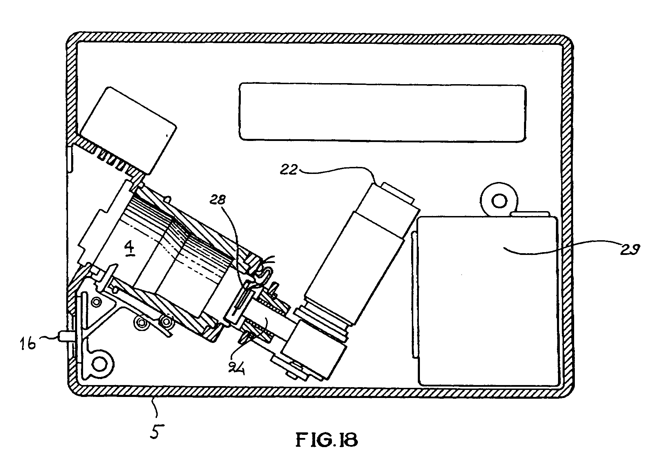

FIG. 18 is a schematic cross-sectional view showing the arrangement of the motor and piston in relation to the housing.

### DETAILED DESCRIPTION:

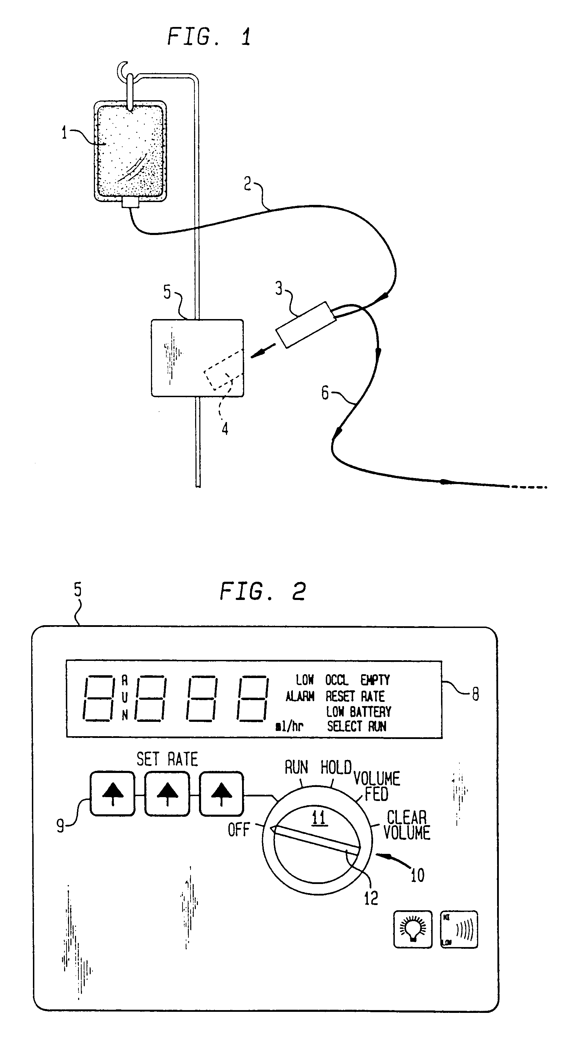

Fig. 1 illustrates the overall pumping system of the present invention. Nutrient flows from a reservoir 1, either hung on an intravenous pole or worn on the patient, down a pump supply tube 2 and into a cassette 3. Preferably, cassette 3 includes a resilient bellows, but other hollow, compressible elements, such as a rubber dome or a spring encased in a collapsible membrane, could be used and are within the scope of the present invention. During operation, cassette 3 is secured within a chamber 4, indicated by dotted lines, within a pump-and-control housing 5. Preferably, chamber 4 is angled about 30° with respect to the pump housing. If supply tube 2 contains any air bubbles they will tend to rise and be purged. After pumping, solution flows from cassette 3 through pump outlet tube 6 into the patient.

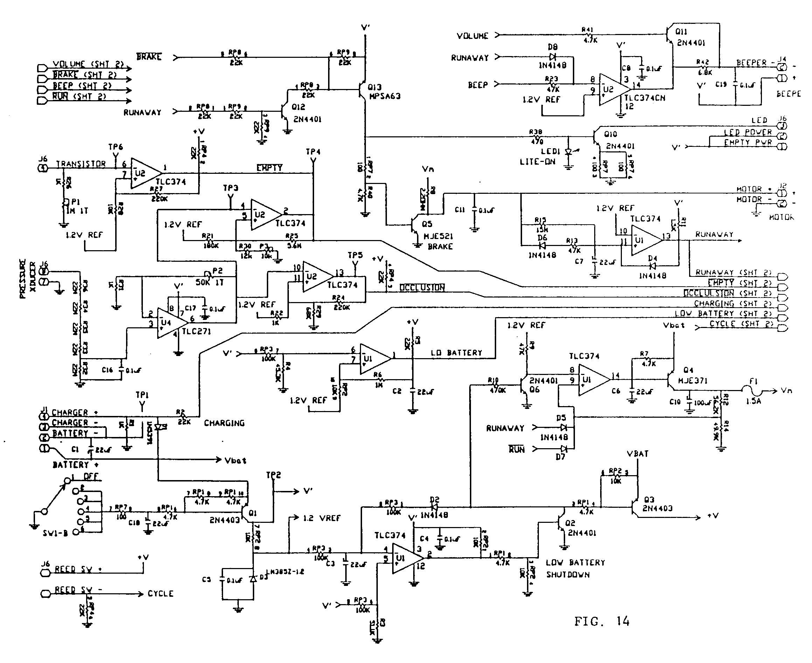

Fig. 2 illustrates the front control panel of the pump housing 5. It features a display window 8, preferably comprising alarm indicators and standard seven-segment displays such as liquid crystal displays (L.C.D) or light-emitting diodes (L.E.D). A suitable LCD is Hamlin model 4717315431. A plurality of buttons 9, preferably membrane switches, permit adjusting the numbers shown in window 8. A novel flip-handle control dial 10 is provided for switching between the modes indicated by the legends on the drawing. A half-spherical recess 11 is provided in the face of the pump housing and permits a disk-shaped handle 12, which is pivotally secured at two of its opposing edges, to be push-pivoted into recess 11. Handle 12 is secured in a dial housing attached to pump housing 5. The dial housing is attached to a multi-position switch, whose six contacts are illustrated at the lower left corner of Fig. 14. The user can place one finger on each face of disk 12 and rotate the disk about a horizontal axis, thereby closing respective switch contacts located, for example, on a printed circuit board (PCB) mounted within housing 5. When the desired setting is reached, disk 12 can be pivoted back flush with the housing surface, leaving no projecting elements to be snagged by an ambulatory patient's clothing, tubing, or the like. This also reduces unauthorized manipulation of the controls.

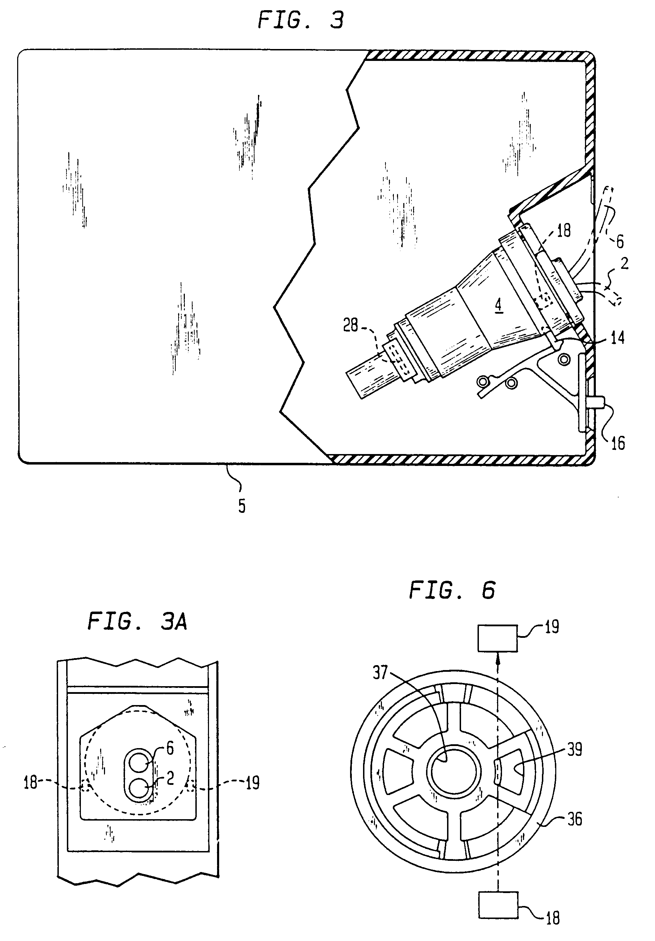

Fig. 3 is a vertical cross-section through pump housing 5, showing a cassette-receiving chamber 4 and other components. The cassette is releasibly secured within chamber 4 by a latch 14, preferably spring-loaded, and released by a sliding handle 16 on the exterior side surface of housing 5. An optical emitter 18, disposed within the wall of a chamber 4 into which cassette 3 is inserted, emits a light beam into one side of cassette 3 and an optical decoder 19 on the opposing side of cassette 3 detects when a chamber 39 within cassette 3 is empty of fluid and causes an alarm to sound. Suitable components 18 and 19 are the TRW Optron #OP240 SLA GaAlAs plastic infrared emitting diode and the TRW Optron #OP550 SLC NPN silicon photo-transistor, both operating at a wavelength of 880 nanometers.

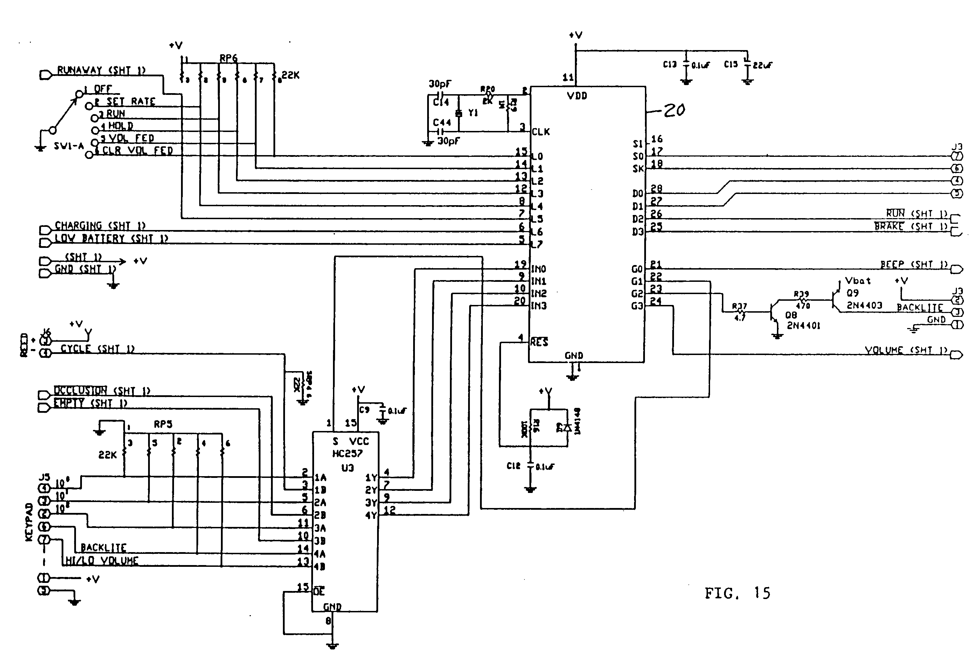

A microprocessor 20, preferably a Thomson/Mostek model ETC 9444N-XYH or a National Semicondcutor model COP444CN, is shown in Fig. 15 and is connected to all electrical elements in the pump housing, and responds to sensor input signals by generating appropriate control output signals in accordance with its program.

In particular, microprocessor 20 generates control pulses for rotation of a motor 22, which rotates a cam and thereby causes a cam follower or piston 24 to compress the bellows portion of cassette 3. Motor 22 is preferably a DC gearmotor such as model 1624E003SP42 + 16/5, 262:1 K297 from MicroMo Electronics. Of course, an equivalent element, such as an eccentric cam, could be used in place of piston 24, with minor modifications. Microprocessor 20 controls window 8's seven-segment display driver 26, which is preferably a National Semiconductor model COP472N-3. Microprocessor 20 also monitors switches 9, control dial 10, optical detector 19, battery energy level, and a pressure sensor 28. Pressure sensor 28 is preferably a piezo-electric disc transducer, such as model PZT-5A #6020 from the Vernitron Piezoelectric division, and detects the pressure between bellows 34 and piston 24.

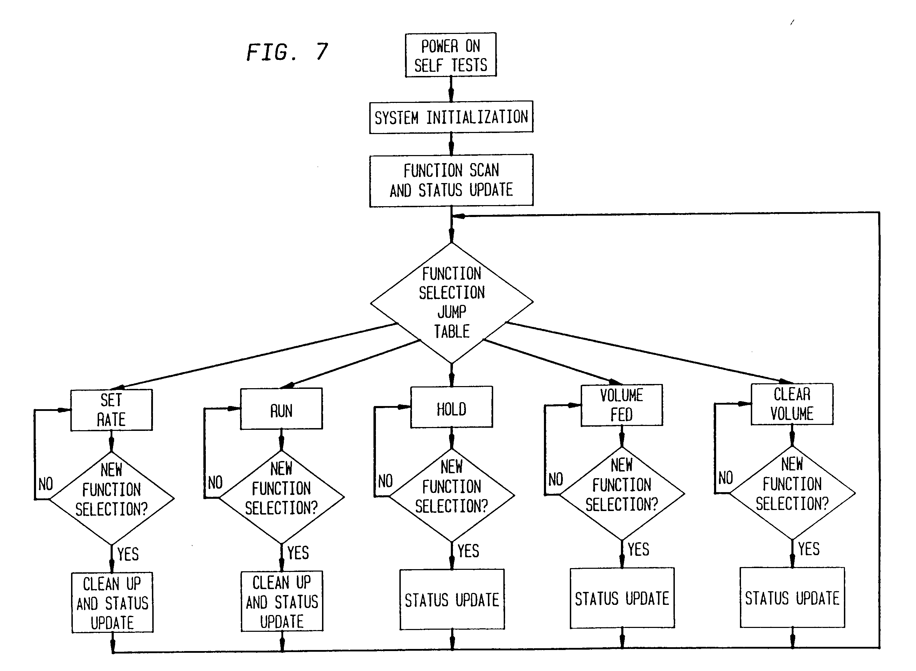

Microprocessor 20 processes the shape of the curve of the variation of pressure with time to detect whether a blockage or break in the tubing has occurred. The system can distinguish the following conditions: (1) an empty cassette-receiving chamber; (2) an upstream blockage or occlusion in supply tube 2; and (3) a downstream blockage or occlusion, such as the patient kinking output tube 6. In the case of an upstream occlusion, the typical bell curve described by the pressure signal from the transducer 28 is shifted some milliseconds later in time with respect to the motor cycle and piston movement.
In the case of a downstream occlusion, the peak of the pressure curve is higher than the normal voltage signal peak of about 1.2 volts. Electrical power is supplied by a battery 29, preferably of the 4-volt variety. The microprocessor's memory preferably includes 128 bytes of RAM (Random Access Memory) and 2 kilobytes of ROM (Read-Only Memory), preferably containing the operating program whose flow charts are shown in Figs. 7-13.

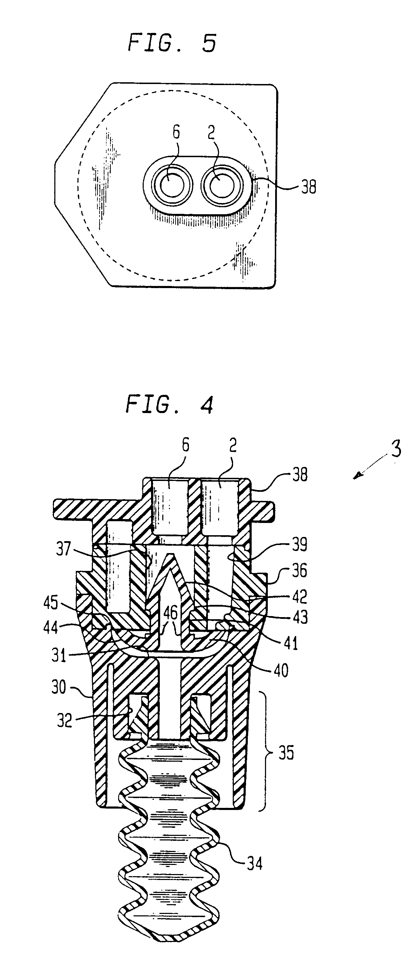

Fig. 4 is a cross-sectional view of cassette 3. The cassette includes a generally cylindrical retainer 30, having at one end a bowl-shaped recess 31, and at the other end an annular chamber 32, into which fits the single open end of a compressible element 34, such as an axially corrugated, generally cylindrical bellows. A tubular protective shroud 35 portion of retainer 30 has a somewhat larger diameter than bellows 34 and surrounds the bellows for a portion of its length. The other end of bellows retainer 30 interfits with, and is secured to, a valve retainer 36 having a generally cylindrical exterior, a central axial passage 37 and an off-center axial passage 39. The interfit may take the form of an annular groove and matching annular rib. Plastic material is used to reduce weight, and other semi-annular recesses may be provided, as shown.

A cassette cap 38, forming apertures for receiving pump supply tube 2 and output tube 6, is secured to the end of valve retainer 36 remote from bellows retainer 30. The periphery of cap 38 preferably has an irregular shape, as shown in Fig. 3A, so that it will fit into housing 5 in only one orientation, thereby preventing erroneous mounting of cassette 3. Preferably, these elements comprise styrene acrylonitrile (SAN) or similar rigid thermoplastic, and are secured together by sonic welding at a frequency of 20,000 Hertz or by adhesives.

Bellows 34 preferably comprises ethyl vinyl acetate (EVA) and is injection blow-molded.

Contained between retainer 30 and valve retainer 36 is a combination valve 40, preferably of a silicone-based material. Valve 40 has a central tubular portion or stem 41, which extends into axial passage 37 of valve retainer 36, containing a duck-bill check valve 42 which prevents backflow of solution away from the patient toward the pump. Valve 42 closes under back-pressure in pump output tube 6 thus prevents backflow, regardless of whether the pump is operating or how the pump, supply reservoir, and tubing are oriented with respect to the patient. Extending from stem 41 is an umbrella valve 44, with its outer peripheral rim 45 pointing away from bellows 34 and toward supply tube 2. Between valves 42 and 44, stem 41 has an outwardly extending annular rib 43, which interfits with a radially inwardly extending annular rib of valve retainer 36.

Operation: Valve 44 opens under a pressure of about 85 inches of water as the "umbrella rim" 45 flexes away from central portion 41, deeper into bowl-shaped recess 31, and permits fluid to flow from tube 2 into the cavity defined by bowl-shaped recess 31 and bellows 34, as bellows 34 expands due to its inherent resiliency. Rib 43 on stem 41 prevents valve 40 from sliding axially. Thereafter, compression of bellows 34 forces a repeatable, metered volume of its contents, preferably about one-third cubic centimeter of fluid, out through central axial aperture 46, duck-bill valve 42 and passage 37 into tube 6. The repetition or pulse rate of these compressions is varied to obtain the desired flow rate.

Valves 42 and 44 are self-actuating and require no external controls, linkages, solenoids, or the like. Their opening and closing is timed exactly by the fluid flow. This substantially improves accuracy, simplifies the construction, increases reliability, and reduces cost. Valve 40 is press-fitted into valve retainer 36. In searching for an appropriate design for valve 40, I recognized that a prototype combination valve shown as model VA 3836 in a catalog from Vernay Laboratories, Inc. of Yellow Springs, Ohio would provide the needed functionality. The basic design of this valve is set forth in U.S. Patent 3,159,176. After some research and development, including adjustment of the "cracking pressure" of the valve, such a valve design was found to be satisfactory. The shape and the pre-loading of the umbrella 44 are important features. Preferably, as set forth above, they are chosen so that valve 44 opens at a pressure somewhere above about 85 inches of water.

Bellows 34 simultaneously serves as metering chamber and performs four functions:
(1) opening inlet valve 44;
(2) sucking in a metered volume of fluid;
(3) providing a return force; and
(4) opening the distal or outlet valve 42 to eject fluid.
The fact that bellows 34 is self-returning means that no attachment of it to the reciprocating element is necessary, unlike prior art syringe pump elements, so that setting up by patients or nurses may be rendered error-free. Bellows 34 is press-fit to bellows retainer 30 and may be further secured by an adhesive, preferably a cyano-acrylic such as Loctite Pyramid 460 Cyanoacrylate, which is water-resistant.

Fig. 5 is an end view of cassette 3, showing that supply tube 2 preferably connects into an off-center aperture to cassette cap 38, while output tube 6 fits centrally. The periphery of cap 38 preferably has an irregular shape, such as the irregular polygon or "arrow" shape shown in Fig. 5, so that it will fit into housing 5 in only one orientation, thereby preventing erroneous mounting of cassette 3 by a relatively unskilled patient or an inadequately trained health care employee. The cassette will not latch into the housing if improperly inserted, and the pressure sensor will cause an alarm to be generated within a predetermined number of motor cycles.

Fig. 6 is a cross-sectional view of the optical system. Two wedge-shaped radial sections of element 36 are cut away or formed with recesses. Emitter 18 directs a beam of light through one of these sections of cassette 3 toward detector 19, which is adjacent the other of these recesses. If fluid is in the path of the beam, the light scatters and is attentuated below a threshold set for the detector signal; if supply tube 2, and thus chamber 39, runs empty, the light is not scattered, detector 19 is triggered, and an alarm indication is generated. Preferably, the word "empty" is illuminated on the LCD window 8 and a beeper sounds.

Fig. 7 is a flow chart of the overall structure of the preferred operating program for microcessor 20 and its associated components. The function scan step detects where the rotating switch shown in Fig. 2 is positioned. The function selection jump table transfers control to the subroutine corresponding to that one of the five functions which has been selected on the control dial.

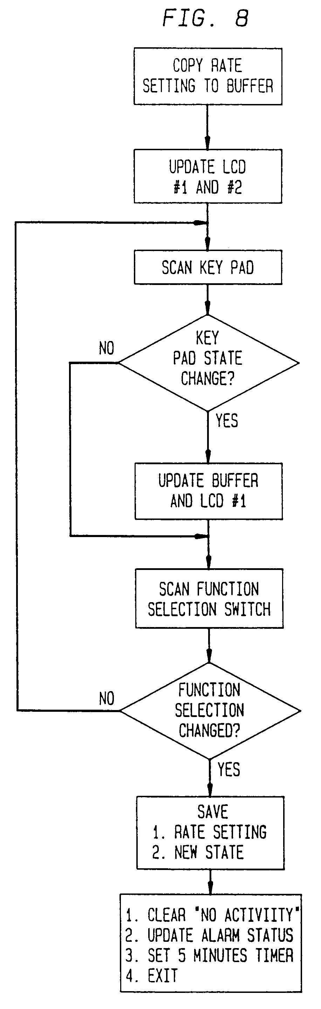

Fig. 8 illustrates the subroutine to SET RATE, that is, to determine how fast the fluid will be pumped, within the preferred operating range of 5-300 milliliters per hour.

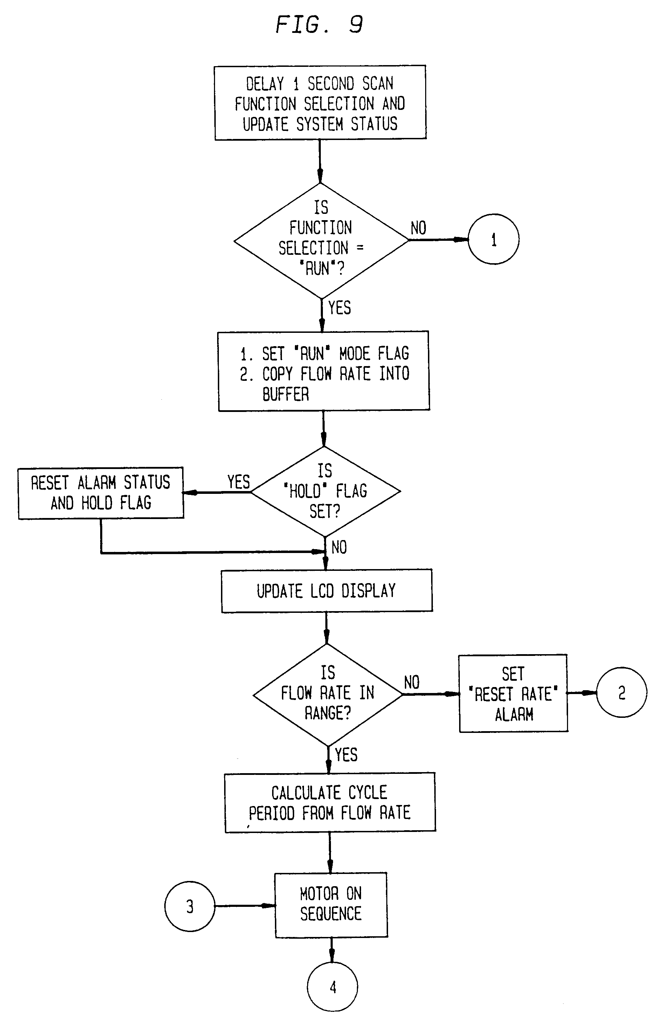

Fig. 9 illustrates the first part of the RUN subroutine, for actually starting pumping at the chosen rate.

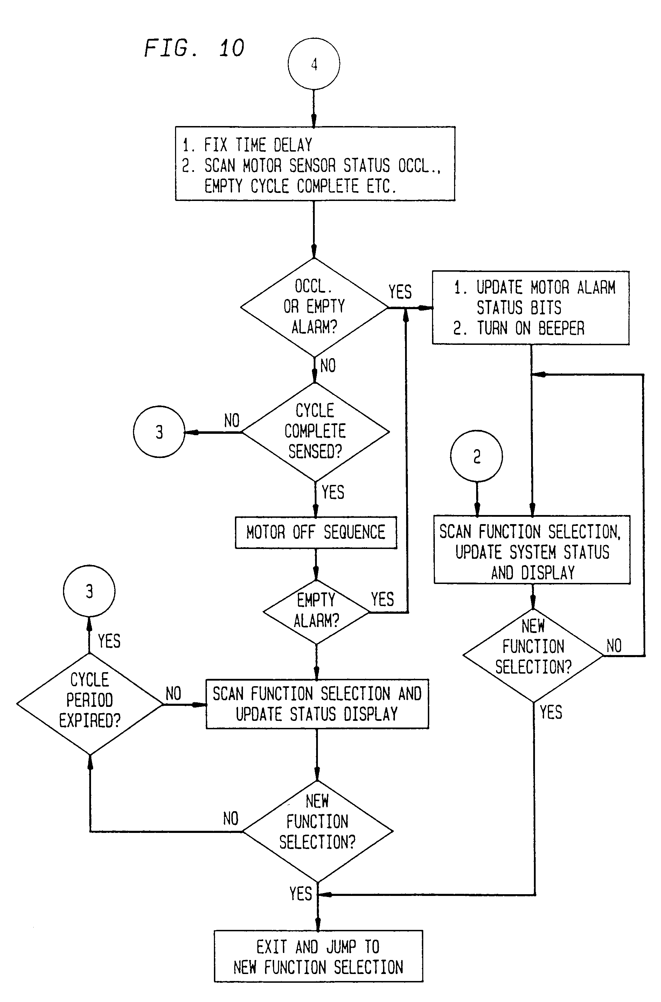

Fig. 10 illustrates the second part of the RUN subroutine, for monitoring sensors to determine if any alarm indications should be generated.

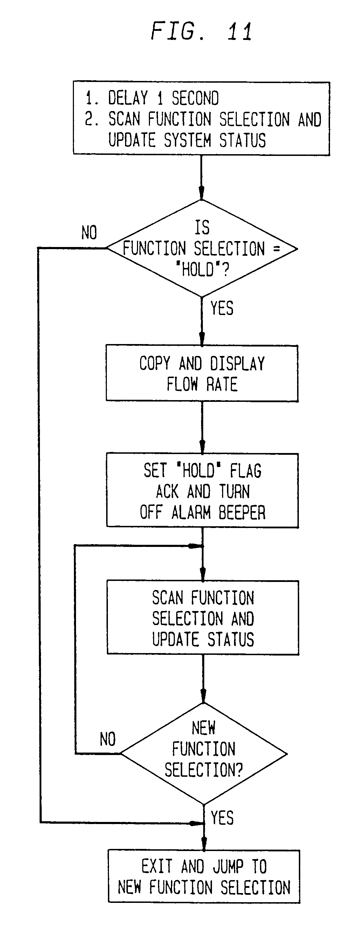

Fig. 11 illustrates the HOLD subroutine, for preserving the data on the volume of fluid fed to the patient while deciding which function or rate to set next.

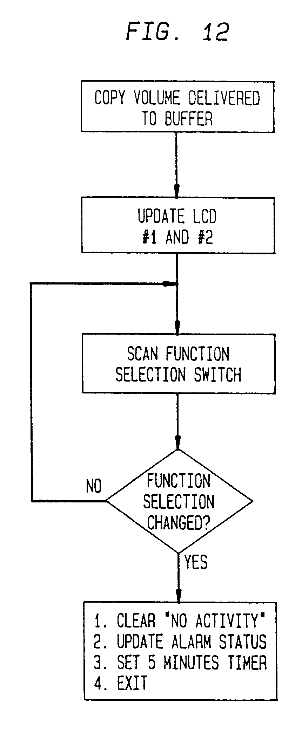

Fig. 12 illustrates the VOLUME FED subroutine. It is noteworthy that this subroutine, as well as the HOLD, SET RATE, and CLEAR VOLUME subroutines, cause an audible alarm if the pump is left unattended for 5 minutes. This prevents patient neglect and preserves battery power.

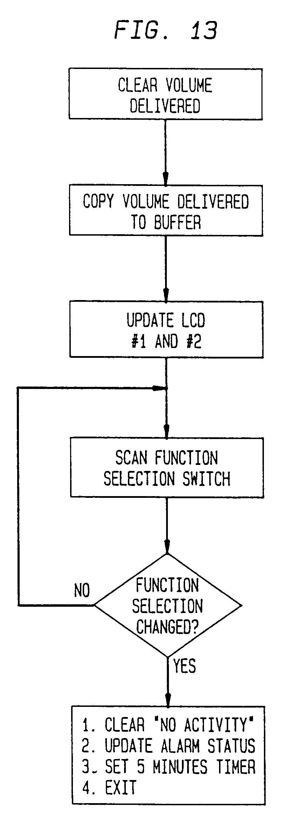

Fig. 13 illustrates the CLEAR VOLUME subroutine, for erasing the data on the volume of fluid fed.

Fig. 14 is sheet 1 of the circuit diagram of the pump circuit. Beginning at lower left, Fig. 14 shows a reed switch which detects the passage of a magnet (not shown) which rotates with the shaft of motor 22. This generates a CYCLE signal which goes into the pin 1B of the multiplexer shown in Fig. 15, and thereby tells the microprocessor 20 when each motor rotation occurs.

Just above the reed switch, the contacts for rotary switch 11-12 are shown. A capacitor C18 and associated resistors serve to "de-bounce" the switch contacts.

Above the rotary switch contacts, the charging circuit, including diode D1, is shown. This circuit supplies V_{bat} for powering other parts of the circuit. A signal goes through resistor R2 to pin L6 (Fig. 15) of microprocessor 20 to indicate when power is coming from the charger in a pole clamp assembly or backpack (not shown) rather than from the battery.

Downstream of diode D1, a circuit including transistor Q1 supplies voltage V′ which supplies all of the comparators and the operational amplifier in the rest of the circuit. This voltage is typically in the range between about 3.65 volts and about 4.5 volts.

Further downstream, in the lower middle portion of Fig. 14, a low-battery shutdown circuit, including a first comparator U1 and transistors Q2 and Q3 acting as switches, shuts down the circuit and latches whenever voltage V′ goes below 3.65 volts. As a safety feature, the circuit cannot be restarted without turning off the pump.

Further to the right in the lower portion of Fig. 14, a circuit including a transistor Q3 supplies a voltage V+ which powers microprocessor 20, the multiplexer, the occlusion detector circuit of Fig. 14, to be described below, and the pull-up resistors shown in Fig. 15.

In the middle right portion of Fig. 14, a resistor R10 connects the low-battery shutdown circuit to a motor voltage regulation circuit including transistors Q6, a second comparator U1, and a power transistor Q4. A fuse F1 is provided at the output for safety. The output of this circuit is a motor supply voltage Vₘ which is preferably about 2.55 volts. Signals applied to the positive input of second comparator U1 shut down the circuit if a "RUNAWAY" condition is detected, i.e. the microprocessor cannot tell when the motor cycle is over.

In the center of Fig. 14, a low-battery warning circuit, including a third comparator U1, detects when battery voltage goes below about 3.95 volts and provides an "early warning" signal to pin L7 (Fig. 15) of microprocessor 20, which can then actuate an alarm before actual shutdown occurs.

### DOWNSTREAM OCCLUSION ALARM ("OCCL")

In the left middle of Fig. 14, a downstream occlusion detector circuit, including an operational amplifier U4 and a fourth comparator U2, determines whether the peak of the pressure transducer 28 output signal exceeds the 1.2 volt reference voltage. If so, this indicates that piston 24 is encountering more-than-usual resistance in compressing bellows 34. A piezo-electric ceramic transducer 28 is mounted at the end of the drive system cam follower or piston 24. During normal pumping, the piston remains in constant contact with the cassette bellows 34, thus producing an electrical potential across the plates of the piezo-electric transducer 28. This signal varies in amplitude with the change in force required to compress the bellows. The transducer signal is fed directly through a high-impedance voltage divider network and a low-pass filter. This reduces the signal level to a usable operating range and eliminates any unwanted noise caused by motion artifact. The transducer signal is fed into amplifier U4, preferably a model TLC271, whose gain is adjusted by a potentiometer P2.
The signal is then compared with a 1.2-volt fixed reference voltage. Under normal operating conditions, the transducer signal falls below the 1.2-volt reference, thus causing the output of fourth comparator U2 to remain HIGH. If a downstream occlusion is present, the amount of force required to compress the bellows 34 increases. This causes the transducer output signal to increase. If the transducer level is greater than the 1.2 volt reference voltage, the output of comparator U2 will trigger LOW. An occlusion signal is supplied to pin 2B (Fig. 15) of the multiplexer for transmission to microprocessor 20, which is preferably programmed to respond when a minimum of 2 motor cycles and/or 45 seconds have passed without relief of the blockage.

### UPSTREAM OCCLUSION ALARM ("OCCL")

Above the aforementioned downstream occlusion detector circuit, Fig. 14 shows a combination upstream occlusion and "cassette not present" detection circuit, including fifth comparator U2. This measures whether the transducer output bell curve exists or is later than usual with respect to the motor cycle. If so, a warning signal is provided to pin 3B (Fig. 15) of the multiplexer for transmission to microprocessor 20. The piezo-electric ceramic transducer is used to detect the presence of an upstream occlusion. During normal pumping, the piston 24 remains in constant contact with bellows 34. When an upstream occlusion, e.g. caused by an obstruction, fluid coagulation, or pinched tubing, occurs, the bellows fails to return to its natural state and to make contact with the piston. As the pump unit commences its next pumping cycle, there is a slight delay between the time the motor turns on and the time piston 24 makes contact with the already-compressed bellows 34. This time delay is monitored by comparator U2, and thence by microprocessor 20.

### CASSETTE NOT PRESENT ALARM ("EMPTY")

During normal pumping, and even when an upstream or downstream occlusion occurs, piston 24 at some point makes contact with bellows 34, generating an output signal from transducer 28. If there is no cassette properly latched into the cassette-receiving chamber, the transducer output signal remains at 0 volts. Therefore, the output of comparator U2 remains HIGH throughout the pumping cycle, which causes microprocessor to generate an "EMPTY" visual and audible alarm within two pumping cycles.

At top left of Fig. 14, a circuit including transistors Q12, Q13, and Q5 shorts the motor terminals at the end of each cycle, as indicated by the reed switch discussed above, thereby causing a counter-electro-motive-force (EMF) to be generated in the motor windings, braking the motor.

### FLUID EMPTY ALARM ("EMPTY")

At top right of Fig. 14, a circuit including transistor Q10 turns on emitter 18 to test whether there is fluid in the cassette. In order to conserve power, emitter 18 is only activated during motor braking. Preferably, a GaAlAs plastic infrared emitter diode and an NPN phototransistor 19 are used in a through-beam configuration (Fig. 6) to detect the lack of fluid in the inlet chamber of the disposable cassette. A potentiometer, P1, controls the collector current of phototransistor 19. This allows for tolerances in the emitter/detector pairs 18, 19. A sixth comparator U2 compares the voltage drop across the phototransistor's emitter resistor to a fixed 1.2-volt reference voltage. The comparator's output is monitored by microprocessor 20. When there is liquid in the inlet chamber of the cassette, the output of comparator U2 is HIGH, the normal operating condition. When the cassette is pumped dry, the output signal goes LOW, triggering an alarm.

Another circuit, including transistor Q11 and a seventh comparator U2, controls actuation and loudness of a beeper, as ordered by VOLUME, RUNAWAY or BEEP signals from microprocessor 20 (see Fig. 15).

Fig. 15 is sheet 2 of the circuit diagram of the pump circuit. As previously noted, microprocessor 2 is preferably a masked Thomson/Mostek model with about 2 kilobytes x 8 bits internal ROM and at least 128 bytes x 4 bits internal RAM. The time-based counter mode is utilized with an internal divide-by-16 counter and an external 2-megahertz crystal to generate a clock signal for an 8-microsecond instruction cycle.

A multiplexer, preferably a CMOS (Complementary Metal Oxide Semiconductor) version of the National Semiconductor model 74HC257 2-data selector, is used to multiplex inputs to the microprocessor.

A restart circuit, comprising a resistor R16, diode D9, and capacitor C12, is connected to the reset pin of the microprocessor.

Output pin G2 of the microprocessor actuates the turning on of a backlight which illuminates the LCD display if the pump is operating on AC power or if the lightbulb logo switch on the front panel (Fig. 2) is pressed. The remainder of Fig. 15 will be self-explanatory to those skilled in the art.

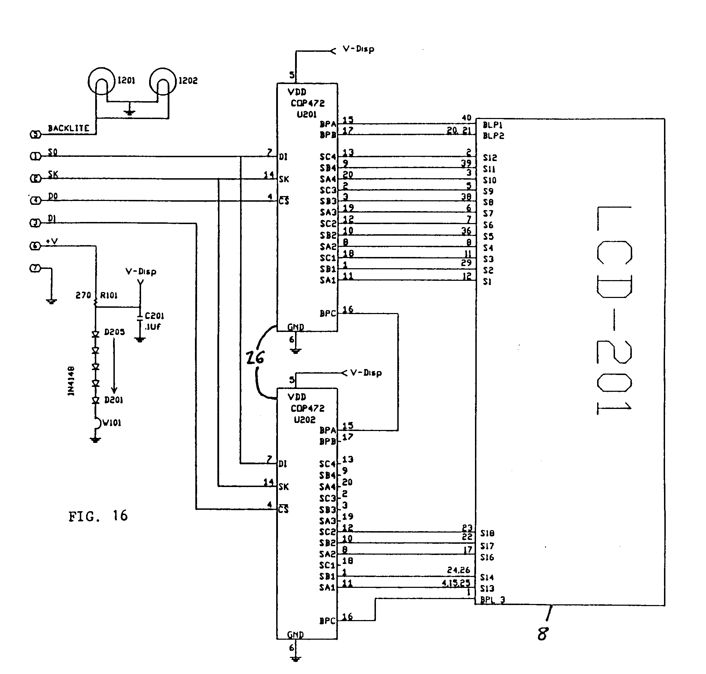

Fig. 16 illustrates the pin-out of the Liquid Crystal Display and its control chips, and their interconnections.

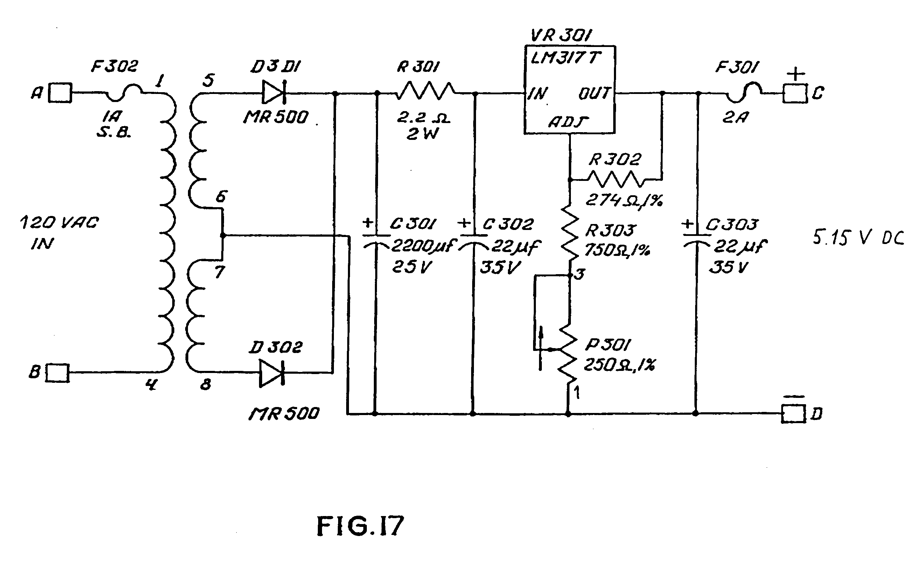

Fig. 17 is a schematic diagram of a power supply for use in operating the pump on A.C. power and in charging the internal rechargeable battery of the pump. Such a power supply is preferably housed in a backpack into which the pump housing can slide sideways. This backpack (not shown) includes a set of contacts which fit into the left side of the pump housing for power transmission purposes. The power supply includes a primary 1-amp slow-blow fuse, a transformer, a pair of diodes D301 and D302, an RC network, a voltage regulator, 1 2-amp output fuse, and a pair of terminals supplying about 5.15 volts direct current for operating the pump or charging its battery.

The backpack preferably has a vertical slot in its back into which a standard hospital pole can be clamped by turning a knob on the side of the backpack and actuating a screw-driven clamp. The power supply circuit is itself supplied with power via an AC cord running from an outlet to the backpack.

Those skilled in the art will appreciate that numerous variations of the cassette and pump described above are possible within the scope of the inventive concept, and that the scope of the invention is not restricted to the embodiments described, but rather is defined by the appended claims.

## Claims

1. A readily sterilizable cassette (3), for use in a pumping device having a supply tube (2), an outlet tube (6) and means (22,24) for intermittently applying pressure to said cassette, comprising
an inlet valve (44) communicating with said supply tube (2);
an outlet check valve (42) communicating with, and preventing backflow from, said outlet tube (6);
a hollow, resilient compressible member (34) having an interior communicating with said inlet and outlet valves; and
a support structure (30,36,38) securing together said tubes, valves and compressible member in operating relation;
characterized in that the valves (42,44) are hydraulically self actuating, in that said compressible member (34) opens said outlet check valve (42) and expels fluid therethrough, in response to pressure from said means (22,24) for intermittently applying pressure, and in that said compressible member (34) resiliently expands in the absence of pressure from said means (22,24) for intermittently applying pressure, thereby opening said hydraulically self-actuating inlet valve (44) and drawing fluid from said supply tube (2).

2. The cassette of claim 1, characterized in that said inlet valve (44) and said outlet valve (42) comprise a unitary element (40).

3. The cassette of claim 1 or 2, characterized in that said compressible member (34) defines a specific volume therewithin which is expelled upon each compression thereof.

4. The cassette of claim 2 or 3, characterized in that said unitary element (40) has a generally umbrella-shaped surface and a generally cylindrical axial stem (41), said outlet valve (42) being disposed in said stem, and an outer rim (45) of said umbrella-shaped surface forming said inlet valve (44).

5. The cassette of one of the claims 1 to 4, characterized in that said compressible member is a bellows (34).

6. The cassette of one of the claims 1 to 5, characterized in that said support structure comprises a bellows retainer (30) and a valve retainer (36) secured together.

7. The cassette of claim 6, characterized in that said retainers are secured together by sonic welding.

8. The cassette of one of the claims 1 to 7, characterized in that said support structure (30,36,38) comprises a rigid thermoplastic material.

9. The cassette of one of the claims 1 to 8, wherein said outlet valve (42) comprises a duck-bill valve.

10. The cassette of one of the claims 1 to 9, characterized in that said inlet valve comprises an umbrella-shaped flexible element (44,45) in a bowl-shaped chamber (31).

11. The cassette of one of the claims 5 to 10, characterized in that said bellows (34) is generally cylindrical and formed with a peripheral rim at an open end thereof, and said bellows retainer (30) is formed with an annular chamber (32) receiving said peripheral rim.

12. The cassette of one of the claims 1 to 11, characterized in that said support structure further comprises a cap (38) formed with cylindrical apertures interfitting with said supply tube (2) and said outlet tube (6).

13. The cassette of one of the claims 1 to 12, characterized in that said cassette includes means (38) having an irregular shape which fits into a pump housing (5) only in a unique, correct orientation.

14. The cassete of claim 13, characterized in that said means (38) having an irregular shape is formed with means for gripping.

15. The cassette of one of the claims 1 to 14, characterized in that it further comprises (Fig. 6) an optical path formed in said cassette and having variable transmission properties, depending upon whether said supply tube (2) contains fluid, thereby permitting external detection (18,19) of fluid supply exhaustion.

16. The cassette of one of the claims 1 to 15, characterized in that said valve retainer (36) is formed with a retaining lip for positioning and latching said cassette within a pump housing.

17. The cassette of one of the claims 1 to 16, characterized in that said compressible member (34) is resiliently self-returning, independently of said means (22,24) for intermittently applying pressure, at a fluid pressure sufficient to actuate said inlet valve (44).

18. A pumping device for fluid media, comprising
a pump housing (5) formed with a cassette-receiving chamber (4);
a cassette (3) according to one of the claims 1 to 17 slidable into said chamber (4);
means (14) for releasibly securing said cassette within said chamber;
means (22, 24) for applying a reciprocating force against said cassette (3), whereas the compressible member (34) of the cassette (3) compresses under force exerted by said force means (22, 24) and resiliently expands following such compression; and
a supply tube (2) and an outlet tube (6) having fluid passage interconnections with said compressible member (34).

19. The pumping device of claim 18, characterized in that said cassette includes an annular valve retainer (36); and whereas
the inlet valve (44) is disposed in said valve retainer (36) between said supply tube (2) and said compressible member (34) and operative to permit fluid flow therebetween while said compressible member is expanding;
the check valve (42) is disposed in said valve retainer (36) between said compressible member (34) and said outlet tube (6), and operative to permit fluid flow therebetween while said compressible member (34) is being compressed, yet preventing backward fluid flow from said outlet tube (6) to said compressible member (34) and said supply tube (2).

20. The pumping device of claim 19, characterized in that said inlet valve (44) comprises
an umbrella-shaped element, formed with a flexible rim (45), disposed in said valve retainer (36) adjacent said supply tube (2), and formed with a central axial aperture which communicates with said outlet tube (6).

21. The pumping device of one of the claims 18 to 20, characterized in that it further comprises
sensors (18, 28) detecting the presence and pressure of fluid within said cassette;
display means (8) and switch means (9, 10) on said pump housing (5); and
a monitoring-and-control circuit (20) metering the volume of fluid pumped through said cassette (3), monitoring output signals from said sensors (18, 28), controlling said reciprocating means in accordance with settings entered via said switch means (9,10), and directing output data to said display means (8).

22. The pumping device of claim 21, characterized in that said monitoring-and-control circuit includes means for detecting blockages or occlusions in said outlet tube (6) by measurement of pressure in said pumping system.

23. The pumping device of claim 22, characterized in that said detecting means comprises a pressure sensor (28) disposed between said force means (22, 24) and said compressible member (34) and generating a pressure signal, and a circuit comparing a peak voltage value of said pressure signal to a reference voltage value.

24. The pumping device of claim 21, characterized in that said monitoring-and-control circuit includes means for detecting blockages or occlusions in said supply tube (2) by measurement of a time interval between a cycle of said force means (22, 24) and a rise in an output signal from said pressure sensor (28).

25. The pumping device of claim 24, characterized in that said detecting means comprises means (D2) indicating a beginning of a cycle of said force means and a circuit comparing an output of said indicating means (D2) and a rise time of said output signal from said pressure sensor (28).

26. The pumping device of one of the claims 21 to 25, characterized in that said monitoring-and-control circuit includes means for detecting exhaustion of fluid in said cassette.

27. The pumping device of claim 26, characterized in that said detecting means comprises an optical emitter (18) disposed in said housing (5) adjacent one side of said cassette (3), an optical detector (19) disposed in said housing adjacent another side of said cassette (3), and a circuit comparing the voltage of the output signal of said optical detector (19) with a reference voltage.

28. The pumping device of one of the claims 21 to 27, characterized in that said monitoring-and-control circuit includes means for detecting presence of said cassette in said chamber (4).

29. The pumping device of one of the claims 25 to 28, characterized in that said detecting means comprises means (D2) indicating a beginning of a cycle of said force means (22, 24) a rotation-sensitive switch on said force means (22, 24) and a circuit comparing output signals of said indicating means (D2) and said rotation-sensitive switch with an output signal from said pressure sensor (28).

30. The pumping device of one of the claims 21 to 29, characterized in that said monitoring-and-control circuit includes a rotation-sensitive switch on said force means and means (Q5) braking said force means after each rotation.

31. A method of operating a pumping device according to one of the claims 18 to 30, further having
means (22, 24) for compressing said cassette (3) or applying a reciprocating force against it;
data entry means (9, 10);
display means (8);
sensor means (19, 28); and
computing means (20) including both fixed and variable memory, said fixed memory containing a stored program, characterized in that it comprises the steps of
initializing said variable memory to predetermined values;
scanning said data entry means (9, 10) to detect any actuation thereof;
selecting and executing a functional subroutine of said stored program in accordance with data, representing a function selection and operating parameters, received from said data entry means (9, 10), thereby changing the operation parameters of the pumping system; and
repeating said scanning step.

32. The method of claim 31, characterized in that one of said subroutines comprises the steps of
reading data, representing a desired pumping rate, from said data entry means (9) into said variable memory;
displaying said pumping rate data on said display means;
scanning said data entry means (9, 10) for any actuation thereof indicative of a new function selection; and
if a function "RUN" is selected, pumping at said desired pumping rate.

33. The method of claim 32, characterized in that one of said subroutines comprises the steps of
calculating from said pumping rate a period between successive cycles for said compressing means (22, 24), and
actuating said compressing means at time intervals equal to said period.

34. The method of claim 31, characterized in that one of said subroutines comprises the steps of
testing whether said sensors indicate any of several predetermined abnormal conditions and, if so, generating at least one alarm indication; and
testing whether a preselected number of cycles of said compressing means (22, 24) have been completed and, if so, turning off said compressing means and generating an indication on said display (8).

35. The method of claim 34, characterized in that said abnormal conditions include at least one of:
a) an occlusion in said supply tube (2);
b) an occlusion in said outlet tube (6);
c) absence of a cassette in said pumping system; and
d) absence of fluid within said cassette (3).

36. The method of claim 31, characterized in that one of said subroutines comprises the steps of
testing whether the function selection "HOLD" has been actuated on said data entry means (10) and, if so, generating a flow rate indication on said display (8); and
turning off any currently activated alarm indications.

37. The method of claim 31, wherein one of said subroutines comprises the steps of
counting a predetermined number of seconds;
testing data received from said data entry means to determine if a new function selection has been actuated and, if not, generating at least one alarm indication.

## Patentansprüche

1. Eine leicht sterilisierbare Kassette (3) zur Verwendung in einem Pumpsystem mit einer Zuleitung (2), einer Auslassleitung (6) und Mitteln (22,24) zur Anlegung intermittierenden Drucks an die besagte Kassette, mit
- einem Einlassventil (44), welches mit der erwähnten Zuleitung (2) in Verbindung steht;
- einem Auslassventil (42), welches mit der erwähnten Auslassleitung (6) in Verbindung steht und ein Zurückströmen von der Auslassleitung (6) verhindert;
- einem hohlen, elastischen und kompressiblen Körper (34), dessen innerer Raum mit den erwähnten Einlass- und Auslassventilen (42,44) in Verbindung steht; und
- einer Supportstruktur (30,36,38), welche die erwähnten Leitungen, Ventile und den kompressiblen Körper zusammenhält,
**dadurch gekennzeichnet, dass**
die Ventile (42,44) dergestalt hydraulisch selbstbetätigend sind, dass der kompressible Körper (34) das Auslassventil (42) als Reaktion auf Druck herrührend von den Mitteln (22,24) zur Anlegung intermittierenden Drucks öffnet und Flüssigkeit durch dasselbe hinaustreibt, und dass der kompressible Körper (34) in der Abwesenheit von Druck herrührend von den Mitteln (22,24) zur Anlegung intermittierenden Drucks elastisch expandiert und dabei das hydraulisch selbstbetätigende Einlassventil (44) öffnet und Flüssigkeit von der Zuleitung (2) anzieht.

2. Kassette gemäss Anspruch 1, dadurch gekennzeichnet, dass das Einlassventil (44) und das Auslassventil (42) ein einheitliches Element (40) darstellen.

3. Kassette gemäss Anspruch 1 oder 2, dadurch gekennzeichnet, dass der kompressible Körper (34) ein spezifisches Volumen umschliesst, welches durch jedes Zusammendrücken hinausgetrieben wird.

4. Kassette gemäss Anspruch 2 oder 3, dadurch gekennzeichnet, dass das einheitliche Element (40) einen im wesentlichen schirmförmigen Oberteil und einen im wesentlichen zylindrischen axialen Schaft (41) besitzt, wobei das Auslassventil (42) in dem erwähnten Schaft (41) angeordnet ist und ein äusserer Rand (45) des schirmförmigen Oberteils das Einlassventil (44) bildet.

5. Kassette gemäss einem der Ansprüche 1 bis 4, dadurch gekennzeichnet, dass der kompressible Teil ein Faltenbalg (34) ist.

6. Kassette gemäss einem der Ansprüche 1 bis 5, dadurch gekennzeichnet, dass die Supportstruktur einen Faltenbalghalter (30) und einen Ventilhalter (36) umfasst, die miteinander verbunden sind.

7. Kassette gemäss Anspruch 6, dadurch gekennzeichnet, dass die erwähnten Halter (30,36) durch Ultrachallschallschweissen miteinander verbunden sind.

8. Kassette gemäss einem der Ansprüche 1 bis 7, dadurch gekennzeichnet, dass die Supportstruktur (30,36,38) aus einem steifen thermoplastischen Material besteht.

9. Kassette gemäss einem der Ansprüche 1 bis 8, dadurch gekennzeichnet, dass das Auslassventil (42) ein Entenschnabel-Ventil ist.

10. Kassette gemäss einem der Ansprüche 1 bis 9, dadurch gekennzeichnet, dass das Einlassventil ein schirmförmiges, flexibles Element (44,45) in einer schalenförmigen Kammer (31) enthält.

11. Kassette gemäss einem der Ansprüche 5 bis 10, dadurch gekennzeichnet, dass der Faltenbalg (34) im wesentlichen zylindrisch ist und an einem offenen Ende einen peripheren Rand aufweist, und dass der Faltenbalghalter (30) eine ringförmige Kammer (32) zur Aufnahme des peripheren Randes besitzt.

12. Kassette gemäss einem der Ansprüche 1 bis 11, dadurch gekennzeichnet, dass die Supportstruktur weiter eine Kappe (38) mit zylindrischen Oeffnungen aufweist, in welche die Zuleitung (2) und die Auslassleitung (6) passen.

13. Kassette gemäss einem der Ansprüche 1 bis 12, dadurch gekennzeichnet, dass die Kassette Mittel (38) mit einer unregelmässigen Form besitzt, welche nur in einer einzigen, korrekten Orientierung in ein Pumpengehäuse (5) passen.

14. Kassette gemäss Anspruch 13, dadurch gekennzeichnet, dass die eine unregelmässige Form aufweisenden Mittel (38) Mittel zum Greifen aufweisen.

15. Kassette gemäss einem der Ansprüche 1 bis 14, dadurch gekennzeichnet, dass in der Kassette ein optischer Pfad ausgebildet ist, dessen Transmissionseigenschaften davon abhängen, ob die Zuleitung (2) Flüssigkeit enthält, sodass eine externe Feststellung (18,19) einer Erschöpfung des Flüssigkeitsvorrates ermöglicht wird.

16. Kassette gemäss einem der Ansprüche 1 bis 15, dadurch gekennzeichnet, dass der Ventilhalter (36) eine Haltelippe aufweist, um die Kassette in einem Pumpengehäuse zu positionieren und einzuklinken.

17. Kassette gemäss einem der Ansprüche 1 bis 11, dadurch gekennzeichnet, dass der kompressible Körper (34) unabhängig von den Mitteln (22,24) zur Anlegung intermittierenden Drucks bei einem Flüssigkeitsdruck, welcher ausreichend ist, um das Einlassventil (44) zu betätigen, elastisch selbst-zurückkehrend ist.

18. Pumpvorrichtung für flüssige Medien, mit einem Pumpengehäuse (5), welches eine Kammer (4) zur Aufnahme einer Kassette ausgebildet hat;
- einer Kassette (3) gemäss einem der Ansprüche 1 bis 17, welche in die Kammer (4) steckbar ist,
- Mitteln (14), um die Kassette in der Kammer (4) lösbar zu befestigen;
- Mitteln (22,24) zur Anlegung einer hin- und herbewegenden Kraft an die Kassette (3), wobei der kompressible Körper (34) der Kassette (3) sich durch die Kraft der erwähnten Kraftmittel (22,24) zusammengedrückt wird und nach dem Zusammendrücken elastisch expandiert; und
- einer Zuleitung (2) und einer Auslassleitung (6) mit Flüssigkeitsdurchlässen zum kompressiblen Körper (34).

19. Pumpvorrichtung gemäss Anspruch 18, dadurch gekennzeichnet, dass die Kassette einen ringförmigen Ventilhalter (36) besitzt; und
- dass das Einlassventil (44) in dem Ventilhalter (36) zwischen der Zuleitung (2) und dem kompressiblen Körper (34) angeordnet ist und so wirkt, dass es zwischen diesen während der Expansion des kompressiblen Körpers einen Flüssigkeitsfluss erlaubt;
- dass das Auslassventil (42 ) in dem Ventilhalter (36) zwischen dem kompressiblen Körper (34) und der Auslassleitung (6) angeordnet ist und so wirkt, dass es zwischen diesen während des Zusammendrückens des kompressiblen Körpers einen Flüssigkeitsfluss erlaubt, aber zugleich einen Flüssigkeitsrückfluss von der Auslassleitung (6) zum kompressiblen Körper (34) und der Zuleitung (2) verhindert.

20. Pumpvorrichtung gemäss Anspruch 19, dadurch gekennzeichnet, dass das Einlassventil (44) ein schirmförmiges Element mit einem flexiblen Rand (45) aufweist, welches in dem zur Zuleitung (2) benachbarten Ventilhalter (36) angeordnet ist und eine zentrale achsiale Oeffnung besitzt, welche mit der Auslassleitung (6) in Verbindung steht.

21. Pumpvorrichtung gemäss einem der Ansprüche 18 bis 20, dadurch gekennzeichnet, dass sie weiter aufweist:
- Sensoren (18,28) zur Feststellung des Vorhandenseins und des Druckes der Flüssigkeit innerhalb der Kassette;
- Anzeigemittel (8) und Eingabemittel (9,10) am Pumpengehäuse (5); und
- einen Ueberwachungs- und Kontrollkreis (20) zur Messung des durch die Kassette (3) gepumpten Flüssigkeitsvolumens, zur Ueberwachung der Ausgangssignale von den Sensoren (18,28), zur Kontrolle der Mittel zum Hin- und Herbewegen in Uebereinstimmung mit Einstellwerten, welche mittels der Eingabemittel (9,10) eingegeben werden, und zum Weiterleiten der Ausgabedaten zu den Anzeigemitteln (8).

22. Pumpvorrichtung gemäss Anspruch 21, dadurch gekennzeichnet, dass der Ueberwachungs- und Kontrollkreis (20) Mittel zur Feststellung von Verstopfungen oder Verschlüssen in der Auslassleitung (6) durch Messung des Druckes in der Pumpvorrichtung aufweist.

23. Pumpvorrichtung gemäss Anspruch 22, dadurch gekennzeichnet, dass die genannten Feststellungsmittel einen Drucksensor (28), welcher zwischen den Kraftmitteln (22,24) und dem kompressiblen Körper (34) angeordnet ist und ein Drucksignal erzeugt, und einen Schaltkreis, welcher einen Spitzenspannungswert des Drucksignals mit einem Referenzspannungssignal vergleicht, aufweisen.

24. Pumpvorrichtung gemäss Anspruch 21, dadurch gekennzeichnet, dass der Ueberwachungs- und Kontrollkreis (20) Mittel zur Feststellung von Verstopfungen oder Verschlüssen in der Auslassleitung (6) besitzt, welche ein Zeitintervall zwischen einem Zyklus der Kraftmittel (22,24) und einem Anstieg des Ausgangssignals vom Drucksensor (28) messen.

25. Pumpvorrichtung gemäss Anspruch 24, dadurch gekennzeichnet, dass die Detektionsmittel
- Mittel (D2), welche den Beginn eines Zyklus der Kraftmittel anzeigen, und
- einen Schaltkreis, welcher ein Ausgangssignal der Anzeigemittel (D2) und die Anstiegszeit des erwähnten Ausgangssignals vom Drucksensor (28) vergleicht, aufweisen.

26. Pumpvorrichtung gemäss einem der Ansprüche 21 bis 25, dadurch gekennzeichnet, dass der Ueberwachungs- und Kontrollkreis Mittel zur Feststellung der Erschöpfung des Flüssigkeitsvorrats der Kassette besitzt.

27. Pumpvorrichtung gemäss Anspruch 26, dadurch gekennzeichnet, dass die Detektionsmittel enthalten:
- eine Lichtquelle (18), welche im Gehäuse (5) an einer Seite der Kassette (3) angeordnet ist,
- einen optischen Sensor (19), welcher im Gehäuse (5) an einer anderen Seite der Kassette (3) angeordnet ist, und
- einen Schaltkreis, welcher die Spannung des Ausgangssignal des optischen Sensors (19) mit einer Referenzspannung vergleicht.

28. Pumpvorrichtung gemäss einem der Ansprüche 21 bis 25, dadurch gekennzeichnet, dass der Ueberwachungs- und Kontrollkreis Mittel enthält, um die Anwesenheit der Kassette in der Kammer (4) festzustellen.

29. Pumpvorrichtung gemäss einem der Ansprüche 25 bis 28, dadurch gekennzeichnet, dass die Detektionsmittel enthalten:
- Mittel (D2) , welche den Beginn eines Zyklus der Kraftmittel (22,24) anzeigen,
- einen drehsensitiven Schalter an den Kraftmitteln (22,24), und
- einen Schaltkreis, welcher die Ausgangssignale der Anzeigemittel (D2) und des drehsensitiven Schalters mit einem Ausgangssignal vom Drucksensor (28) vergleicht.

30. Pumpvorrichtung gemäss einem der Ansprüche 21 bis 29, dadurch gekennzeichnet, dass der Ueberwachungs- und Kontrollkreis einen drehsensitiven Schalter an den Kraftmitteln und Mittel (Q5) enthält, welche die Kraftmittel nach jeder Rotation bremsen.

31. Verfahren, um eine Pumpvorrichtung gemäss einem der Ansprüche 18 bis 30 zu betreiben, wobei die Pumpvorrichtung weiter enthält:
- Mittel (22,24) zum Zusammendrücken der Kassette (3) oder zum Anlegen einer hin- und herbewegenden Kraft an diese,
- Dateneingabemittel (9,10),
- Sensormittel (19,28); und
- Rechnermittel (20) mit festem und variablem Speicher, wobei der feste Speicher ein Programm gespeichert hat,
dadurch gekennzeichnet, dass das Verfahren die folgenden
Schritte aufweist:
- Initialisieren des variablen Speichers auf vorbestimmte Werte;
- Abtasten der Dateneingabemittel (9,10), um eine Betätigung derselben festzustellen,
- Auswahl und Ausführung eines funktionellen Unterprogramms des gespeicherten Programms in Uebereinstimmung mit Daten, welche durch eine Funktionsauswahl und durch Arbeitsparameter, welche von den Dateneingabemitteln (9,10) erhalten werden, zustandekommen, wobei die Betriebsparameter der Pumpvorrichtung geändert werden; und
- Wiederholen des Abtastens.

32. Verfahren gemäss Anspruch 31, dadurch gekennzeichnet, dass eines der Unterprogramme die folgenden Schritte aufweist:
- Einlesen der Daten, welche eine gewünschte Pumpgeschwindigkeit darstellen, von den Dateneingabemitteln (9) in den variablen Speicher;
- Anzeigen der Pumpgeschwindigkeit an den Anzeigemitteln;
- Abtasten der Dateneingabemittel (9,10), um eine Betätigung derselben festzustellen, was eine neue Funktionswahl anzeigt; und, falls eine Funktion "LAUFEN" gewählt ist,
- Pumpen mit der gewünschten Pumpgeschwindigkeit.

33. Verfahren gemäss Anspruch 32, dadurch gekennzeichnet, dass eines der Unterprogramme die folgenden Schritte aufweist:
- Berechnen der Zeitperiode zwischen aufeinanderfolgenden Zyklen aufgrund der Pumpgeschwindigkeit für die Kompressionsmittel (22,24); und
- Betätigen der Kompressionsmittel mit einem Zeitintervall, welches der Zeitperiode entspricht.

34. Verfahren gemäss Anspruch 31, dadurch gekennzeichnet, dass eines der Unterprogramme die folgenden Schritte aufweist:
- Testen, ob die Sensoren irgendeinen von mehreren vorbestimmten, abnormalen Zuständen anzeigen und, falls dies der Fall ist, wenigstens eine Alarmanzeige erzeugen; und
- Testen, ob eine vorgewählte Anzahl Zyklen der Kompressionsmittel (22,24) ausgeführt ist, und, falls dies der Fall ist, Abschalten der Kompressionsmittel und Erzeugung einer Anzeige an den Anzeigemitteln (8).

35. Verfahren gemäss Anspruch 34, dadurch gekennzeichnet, dass die abnormalen Zustände wenigstens einen der nachfolgenden einschliesst:
- Verschluss in der Zuleitung (2);
- Verschluss in der Auslassleitung (6);
- Fehlen der Kassette in der Pumpvorrichtung;
- Fehlen von Flüssigkeit in der Kassette (3).

36. Verfahren gemäss Anspruch 31, dadurch gekennzeichnet, dass eines der Unterprogramme die folgenden Schritte aufweist:
- Testen, ob die Funktionswahl "HALTEN" an den Dateneingabemitteln betätigt wurde und, falls dies der Fall ist, eine Flussgeschwindigkeitsanzeige an den Anzeigemitteln (8) erzeugt; und
- Abschalten von irgendwelchen momentan akivierten Alarmanzeigen.

37. Verfahren gemäss Anspruch 31, dadurch gekennzeichnet, dass eines der Unterprogramme die folgenden Schritte aufweist:
- Zählen einer vorbestimmten Anzahl Sekunden;
- Prüfen der von den Dateneingabemitteln herrührenden Daten, um festzustellen, ob eine neue Funktionswahl betätigt wurde, und, falls dies nicht der Fall war, wenigsten eine Alarmanzeige erzeugt.

## Revendications

1. Cartouche facilement stérilisable (3), destinée à être utilisée dans un dispositif de pompage qui comporte un tube d'alimentation (2), un tube d'écoulement (6) et des moyens (22, 24) pour appliquer par intermittence une pression à ladite cartouche, comprenant
un clapet d'admission (44) en communication avec ledit tube d'alimentation (2);
un clapet de refoulement anti-retour (42) en communication avec ledit tube d'écoulement (6) et s'opposant à un retour de courant à partir de celui-ci;
un organe creux, élastiquement compressible (34) dont le volume intérieur est en communication avec lesdits clapets d'admission et de refoulement; et
une structure de support (30, 36, 38) qui fixe ensemble lesdits tubes, clapets et organe compressible en position de fonctionnement;
caractérisée en ce que les clapets (42, 44) sont auto-actionnables hydrauliquement, en ce que ledit organe compressible (34) ouvre ledit clapet de refoulement anti-retour (42) et expulse du liquide à travers celle-ci, en réponse à une pression produite par lesdits moyens (22, 24) d'application intermittente de pression, et en ce que ledit organe compressible (34) s'étend élastiquement, en l'absence de pression produite par lesdits moyens (22, 24) d'application intermittente de pression, en ouvrant ainsi ledit clapet d'admission (44) auto-actionnable hydrauliquement et en aspirant du liquide depuis ledit tube d'alimentation (2).

2. Cartouche selon la revendication 1, caractérisée en ce que ledit clapet d'admission (44) et ledit clapet de refoulement (42) constituent un élément unitaire (40).

3. Cartouche selon la revendication 1 ou 2, caractérisée en ce que ledit organe compressible (34) définit intérieurement un volume spécifique qui est expulsé à chaque compression de cet organe.

4. Cartouche selon la revendication 2 ou 3, caractérisée en ce que ledit élément unitaire (40) comporte une surface en forme générale de parasol et une tige axiale (41) de forme générale cylindrique, ledit clapet de refoulement (42) étant disposé dans ladite tige, et un bord extérieur (45) de ladite surface en forme de parasol formant ledit clapet d'admission (44).

5. Cartouche selon l'une quelconque des revendications 1 à 4, caractérisée en ce que ledit organe compressible est un soufflet (34).

6. Cartouche selon l'une quelconque des revendications 1 à 5, caractérisée en ce que ladite structure de support comprend un élément (30) de montage du soufflet et un élément (36) de montage des clapets, qui sont fixés l'un à l'autre.

7. Cartouche selon la revendication 6, caractérisée en ce que lesdits éléments de montage sont fixés l'un à l'autre par soudage par ultrasons.

8. Cartouche selon l'une quelconque des revendications 1 à 7, caractérisée en ce que ladite structure de support (30, 36, 38) est faite d'une matière thermoplastique rigide.

9. Cartouche selon l'une quelconque des revendications 1 à 8, dans laquelle ledit clapet de refoulement (42) est constitué par un clapet en bec de canard.

10. Cartouche selon l'une quelconque des revendications 1 à 9, caractérisée en ce que ledit clapet d'admission comprend un élément flexible (44, 45) en forme de parasol dans une chambre (31) en forme de bol.

11. Cartouche selon l'une quelconque des revendications 5 à 10, caractérisée en ce que ledit soufflet (34) est de forme générale cylindrique et comporte un bourrelet périphérique à son extrémité ouverte, et en ce qu'il est formé, dans ledit élément (30) de montage du soufflet, une chambre annulaire (32) qui reçoit ce bourrelet périphérique.

12. Cartouche selon l'une quelconque des revendications 1 à 11, caractérisée en ce que ladite structure de support comprend en outre une coiffe (38) dans laquelle dont formées des ouvertures cylindriques où s'adaptent ledit tube d'alimentation (2) et ledit tube d'écoulement (6).

13. Cartouche selon l'une quelconque des revendications 1 à 12, caractérisée en ce qu'elle comprend des moyens (38) de forme irrégulière qui ne s'adaptent dans un carter (5) de pompe que dans une seule orientation correcte.

14. Cartouche selon la revendication 13, caractérisée en ce que lesdits moyens (38) de forme irrégulère comportent des moyens de bridage.

15. Cartouche selon l'une quelconque des revendications 1 à 14, caractérisée en ce qu'il y est en outre formé (fig. 6) un trajet optique qui a des propriétés de transmission variables selon que ledit tube d'alimentation (2) contient ou ne contient pas de liquide, ce qui permet une détection externe (18, 19) de l'épuisement de l'alimentation en liquide.

16. Cartouche selon l'une quelconque des revendications 1 à 15, caractérisée en ce qu'il est formé, dans ledit élément (36) de montage des clapets, une lèvre de retenue pour positionner et verrouiller ladite cartouche dans un carter de pompe.

17. Cartouche selon l'une quelconque des revendications 1 à 16, caractérisée en ce que ledit organe compressible (34) se remet spontanément en l'état initial par élasticité, indépendamment desdits moyens (22, 24) d'application de pression intermittente, lorsque la pression du liquide est suffisante pour actionner ledit clapet d'admission (44).

18. Dispositif de pompage pour produits liquides, comprenant
un carter de pompe (5) dans lequel est formée une chambre (4) de logement de cartouche;
une cartouche (3) selon l'une quelconque des revendications 1 à 7, pouvant être insérée dans ladite chambre (4);
des moyens (14) pour fixer ladite cartouche dans ladite chambre de façon détachable;
des moyens (22, 24) pour appliquer une force à mouvement de va-et-vient contre ladite cartouche (3), de telle sorte que l'organe compressible (34) de la cartouche (3) soit comprimé sous l'effet de la force exercée par lesdits moyens (22, 24) générateurs de force et qu'il s'étende par élasticité à la suite d'une telle compression; et
un tube d'alimentation (2) et un tube d'écoulement (6) raccordés audit organe compressible (34) par des passages de liquide,

19. Dispositif de pompage selon la revendication 18, caractérisé en ce que ladite cartouche comporte un élément annulaire (36) de montage des clapets,
en ce que le clapet d'admission (44) est disposé dans cet élément de montage (36) entre ledit tube d'alimentation (2) et ledit organe compressible (34) et agit de manière à permettre le passage de liquide entre ceux-ci pendant que ledit organe compressible s'étend; et
en ce que le clapet anti-retour (42) étant disposé dans ledit élément (36) de montage des clapets entre ledit organe compressible (34) et ledit tube d'écoulement (6), et agit de manière à permettre le passage de liquide entre ceux-ci pendant que ledit organe compressible (34) est comprimé, tout en empêchant un reflux de liquide à partir dudit tube d'écoulement (6) vers ledit organe compressible (34) et ledit tube d'alimentation (2).

20. Dispositif de pompage selon la revendication 19, caractérisé en ce que ledit clapet d'admission (44) comprend
un élément en forme de parasol, qui comporte un bord flexible (45), est disposé dans ledit élément (36) de montage des clapets à proximité dudit tube d'alimentation (2), et dans lequel est formée une ouverture axiale centrale qui est en communication avec ledit tube d'écoulement (6).

21. Dispositif de pompage selon l'une quelconque des revendications 18 à 20, caractérisé en ce qu'il comprend en outre
des détecteurs (18, 28) qui détectent la présence et la pression d'un liquide dans la cartouche;
des moyens d'affichage (8) et des moyens de commutation (9, 10) sur ledit carter de pompe (5); et
un circuit de surveillance et de commande (20) qui mesure le volume de liquide pompé à travers ladite cartouche (3), surveille les signaux de sortie desdits détecteurs (18, 28), commande lesdits moyens générateurs de force à mouvement de va-et-vient selon des valeurs de réglage introduites à l'aide des moyens commutateurs (9, 10) et dirige des données de sortie vers lesdits moyens d'affichage (8).

22. Dispositif de pompage selon la revendication 21, caractérisé en ce que ledit circuit de surveillance et de commande comprend des moyens pour détecter des blocages ou des occlusions dans ledit tube d'écoulement (6) par mesure de la pression dans ledit dispositif de pompage.

23. Dispositif de pompage selon la revendication 22, caractérisé en ce que lesdits moyens de détection comprennent un capteur de pression (28) disposé entre lesdits moyens générateurs de force (22, 24) et ledit organe compressible (34) et émettant un signal de pression, et un circuit qui compare une valeur de tension de crête dudit signal de pression à une valeur de tension de référence.

24. Dispositif de pompage selon la revendication 21, caractérisé en ce que ledit circuit de surveillance et de commande comprend des moyens pour détecter des blocages ou des occlusions dans ledit tube d'alimentation (2) par mesure de l'intervalle de temps entre un cycle desdits moyens générateurs de force (22, 24) et la montée d'un signal de sortie dudit capteur de pression (28).

25. Dispositif de pompage selon la revendication 24, caractérisé en ce que lesdits moyens de détection comprennent des moyens (D2) qui indiquent le début d'un cycle desdits moyens générateurs de force, et un circuit qui compare le signal de sortie desdits moyens indicateurs (D2) et le temps de montée dudit signal de sortie dudit capteur de pression (28).

26. Dispositif de pompage selon l'une quelconque des revendications 21 à 25, caractérisé en ce que ledit circuit de surveillance et de commande comprend des moyens pour détecter l'épuisement de liquide dans ladite cartouche.

27. Dispositif de pompage selon la revendication 26, caractérisé en ce que lesdits moyens de détection comprennent un émetteur optique (18) disposé dans ledit carter (5) prés d'un côté de ladite cartouche (3), un détecteur optique (19) disposé dans ledit carter près d'un autre côté de ladite cartouche (3), et un circuit qui compare la tension du signal de sortie dudit détecteur optique (19) à une tension de référence.

28. Dispositif de pompage selon l'une quelconque des revendications 21 à 27, caractérisé en ce que ledit circuit de surveillance et de commande comprend des moyens pour détecter la présence de ladite cartouche dans ladite chambre (4).

29. Dispositif de pompage selon l'une quelconque des revendications 25 à 28, caractérisé en ce que lesdits moyens de détection comprennent des moyens (D2) qui indiquent le début d'un cycle desdits moyens générateurs de force (22, 24), un comutateur sensible à une rotation, disposé sur lesdits moyens générateurs de force (22, 24), et un circuit qui compare les signaux de sortie desdits moyens indicateurs (D2) et dudit commutateur sensible à une rotation à un signal de sortie dudit capteur de pression (28).

30. Dispositif de pompage selon l'une quelconque des revendications 21 à 29, caractérisé en ce que ledit circuit de surveillance et de commande comprend un commutateur sensible à une rotation, disposé sur lesdits moyens générateurs de force, et des moyens (Q5) qui freinent lesdits moyens générateurs de force après chaque rotation.

31. Procédé pour faire fonctionner un dispositif de pompage selon l'une quelconque des revendications 18 à 30, comportant en outre
des moyens (22, 24) pour comprimer ladite cartouche (3) ou lui appliquer une force à mouvement de va-et-vient;
des moyens d'introduction de données (9, 10);
des moyens d'affichage (8);
des moyens détecteurs (19, 28); et
des moyens calculateurs (20) qui comprennent une mémoire fixe et une mémoire variable, ladite mémoire fixe contenant un programme enregistré, caractérisé en ce qu'il comprend les étapes consistant
à initialiser ladite mémoire variable à des valeurs prédéterminées;
à explorer lesdits moyens d'introduction de données (9, 10) pour détecter une éventuelle mise en fonction de ceux-ci;
à sélectionner et à exécuter un sous-programme fonctionnel dudit programme enregistré selon des données qui représentent une sélection de fonction et des paramètres d'exploitation et qui sont reçues desdits moyens d'introduction de données (9, 10), de manière à modifier ainsi les paramètres de fonctionnement du dispositif de pompage; et
à répéter ladite étape d'exploration.

32. Procédé selon la revendication 31, caractérisé en ce que l'un desdits sous-programmes comprend les étapes consistant
à extraire des données représentant un débit de pompage désiré à partir desdits moyens d'introduction de données (9) et à les enregistrer dans ladite mémoire variable;
à visualiser lesdites données de débit de pompage sur lesdits moyens d'affichage;
à explorer lesdits moyens d'introduction de données (9, 10) à la recherche d'une éventuelle mise en fonction de ceux-ci indiquant une nouvelle sélection de fonction; et
si la fonction "MARCHE" est sélectionnée, à pomper avec ledit débit de pompage désiré.

33. Procédé selon la revendication 32, caractérisé en ce que l'un desdits sous-programmes comprend les étapes consistant
à calculer, à partir dudit débit de pompage, une période entre des cycles successifs desdits moyens de compression (22, 24); et
à actionner lesdits moyens de compression à des intervalles de temps égaux à ladite période.

34. Procédé selon la revendication 31, caractérisé en ce que l'un des sous-programmes comprend les étapes consistant
à tester si lesdits détecteurs indiquent l'une quelconque parmi plusieurs conditions anormales prédéterminées et, si tel est le cas, à émettre au moins une indication d'alarme; et
à tester si un nombre préalablement choisi de cycles desdits moyens de compression (22, 24) a été effectué et, si tel est le cas, à mettre à l'arrêt lesdits moyens de compression et à produire une indication sur lesdits moyens d'affichage (8).

35. Procédé selon la revendication 34, caractérisé en ce que lesdites conditions anormales comprennent l'un au moins des incidents suivants:
a) occlusion dans ledit tube d'alimentation (2):
b) occlusion dans ledit tube d'écoulement (6);
c) absence de cartouche dans ledit dispositif de pompage;
et
d) absence de liquide dans ladite cartouche (3).

36. Procédé selon la revendication 31, caractérisé en ce que l'un desdits sous-programmes comprend les étapes consistant
à tester si la sélection de la fonction "MAINTIEN" a été effectuée à l'aide desdits moyens d'introduction de données (10) et, si tel est le cas, à produire une indication de débit sur lesdits moyens d'affichage (8); et
à supprimer d'éventuelles indications d'alarme présentement activées.

37. Procédé selon la revendication 31, dans lequel l'un desdits sous-programmes comprend les étapes consistant
à compter un nombre prédéterminé de secondes;
à tester les données reçues desdits moyens d'introduction de données pour déterminer si une nouvelle sélection de fonction a été faite et, si tel n'est pas le cas, à produire au moins une indication d'alarme.
